# FASCICULE DE BREVET EUROPEEN

(11) **EP 4 413 963 B1**
(45) Date de publication et mention de la délivrance du brevet: **05.08.2026**
(21) Numéro de dépôt: 24156205.7
(22) Date de dépôt: 07.02.2024
(51) Int. Cl.: A61F 13/15, A61F 13/496, A61F 13/84, A41B 9/04

(54) **CULOTTE MENSTRUELLE CHAUFFANTE**
WÄRMENDE MENSTRUATIONSHOSE
HEATED MENSTRUAL PANT

(30) Priorité: 10.02.2023 FR 2301259
(43) Date de publication de la demande: 14.08.2024
(73) Titulaire: Frati, Albane, 75013 Paris (FR)
(72) Inventeur: FRATI, Albane, 75013 Paris (FR)
(74) Mandataire: Eidelsberg, Olivier Nathan

(56) Documents cités:
- CN-A- 104 055 618
- CN-U- 204 763 437
- CN-Y- 201 119 423
- US-A1- 2009 118 574

## Description

La présente invention se rapporte à une culotte menstruelle pour soulager la femme des dysménorrhées.

Les dysménorrhées sont des douleurs de règles plus ou moins intenses apparaissant après les premières règles. Il est connu de traiter de manière symptomatique ces douleurs par la chaleur, par exemple par un bain chaud, ou de manière plus ciblée en appliquant de la chaleur au niveau du pelvis et de la zone lombaire basse.

Il est courant pour les femmes souffrant de dysménorrhées de s'appliquer une bouillotte sur le ventre pour soulager la douleur. L'ergonomie de la bouillotte ne permet pas de maintenir une activité normale et une vie active, l'utilisation se faisant allongée avec une bouillotte difficilement transportable. Ces bouillottes classiques présentent également des risques de brûlure. De plus, les bouillottes sont voyantes et les femmes peuvent refuser de les utiliser en public, notamment sur leur lieu de travail. Il existe des patchs chauffants ou des patchs TENS électrostimulants à usage unique et à durée de chauffe limitée. Ces patchs sont appliqués aléatoirement sur la culotte, ce qui provoque un danger de mauvaise utilisation et limite leur efficacité. Ils ne permettent pas le traitement des douleurs dans la zone postérieure lombaire, fréquentes des dysménorrhées. De plus, ces patchs à usage unique ont un coût important lorsqu'ils sont utilisés régulièrement. On connaît également des culottes comportant une poche de type kangourou dans laquelle on peut insérer une chaufferette. Ces chaufferettes restent mobiles dans la poche et sont mal adaptées à la zone à traiter. Elles ne permettent pas non plus de traiter la zone postérieure du corps et ont des durées de chauffage très courtes. Il existe également des culottes menstruelles en un matériau absorbant pour absorber les règles. Ces culottes ont une durée de vie courte en raison des nombreux lavages nécessaires.

De CN204763437U, on connaît également de répondre à ces souffrances par une culotte à laquelle sont fixés un fil électrique chauffant et des packs magnétiques de médecine chinoise.

De CN201119423Y, on connaît également de fixer des éléments chauffant sur la face avant de la culotte, en partie haute, ces éléments étant alimenté par une batterie.

De CN205286665U, on connait une culotte à laquelle sont fixés des chaufferettes et autres pierres comme la tourmaline.

De CN205912909, il est connu de fixer des fils chauffants sur le tissu formant la face avant ou antérieure de la culotte.

Enfin, de KR 10206468581, il est connu de réaliser une culotte en un tissu tissé à partir de fils chauffants, les fils chauffants étant des fils textiles recouverts d'un polymère conducteur, notamment par dépôt en phase vapeur.

Ces culottes ont une faible durée de vie, notamment en raison du fait que les éléments chauffants fixés à la culotte ont tendance à s'en détacher au cours du temps, notamment en cas d'une utilisation répétée. En outre, ces culottes ne sont pas en un matériau absorbant au niveau de leur entrejambe, ce qui les rend peu adaptées à une utilisation pendant les règles. Quant à la culotte réalisée en tissu chauffant, elle résiste mal au lavage, les tissés en fils chauffants ayant tendance à se dégrader et à perdre de leur fonction chauffante au bout d'un certain nombre de lavages, notamment lavages à hautes températures. De même, les batteries intégrées à ces culottes de l'art antérieur résistent mal aux lavages répétés.

L'invention vise à résoudre les problèmes cités précédemment par une culotte menstruelle suivant la revendication 1, des perfectionnements et modes de réalisation préférés étant visés aux sous revendications.

La culotte menstruelle chauffe ainsi l'utilisatrice pendant une longue durée, tout en étant discret. Ce dispositif est réutilisable avec une longue durée de vie, l'entre-jambe de la culotte faisant office de protection hygiénique en absorbant les règles et pouvant être remplacé par un autre entre-jambe et/ou être lavé indépendamment du reste de la culotte, notamment plus fréquemment, sans risquer de détériorer ou abimer le dispositif électrique du tissu chauffant du reste de la culotte.

Une partie de la face antérieure peut être en tissu chauffant. De préférence, la partie en tissu chauffant de la face antérieure est destinée à être en face du pelvis de la personne portant la culotte. On traite ainsi les douleurs du pelvis de l'utilisatrice.

Une partie de la face postérieure peut être en tissu chauffant. De préférence, la partie en tissu chauffant de la face postérieure est destinée à être en face de la zone lombaire basse de la personne portant la culotte. On traite ainsi à la fois les douleurs du pelvis et de la zone lombaire basse de l'utilisatrice.

À titre d'exemple, on décrit maintenant un mode de réalisation préféré de l'invention en se reportant aux dessins, dans lesquels :

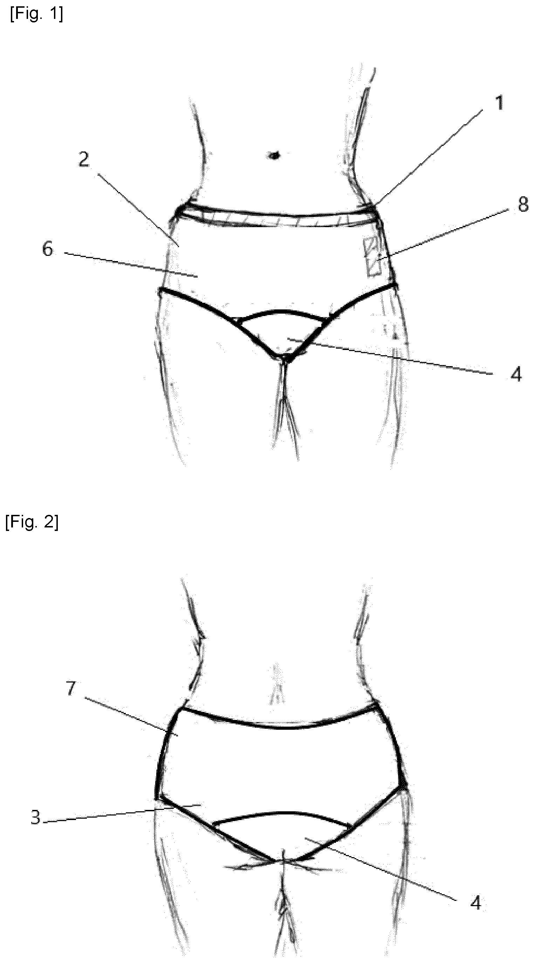

la figure 1 est une vue de face de la face antérieure de la culotte suivant l'invention, à l'état porté ;
la figure 2 est une vue de face de la face postérieure de la culotte suivant l'invention, à l'état porté ;
la figure 3 est une vue en perspective de face de la partie formant ceinture de la culotte des figures 1 et 2;
la figure 4 est une vue de face de la partie formant l'entre-jambe de la culotte des figures 1 et 2 ; et

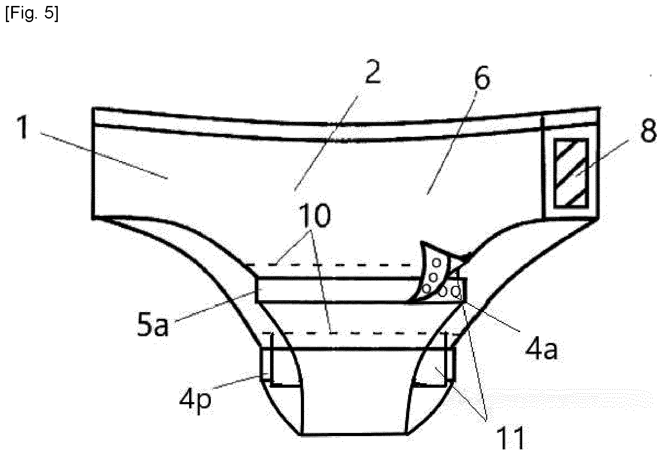

la figure 5 est une vue en perspective de la culotte des figures 1 et 2, avec l'entre-jambe en partie détaché de la face postérieure.

La culotte 1 comporte une partie 20 formant ceinture ayant une face 2 antérieure et une face 3 postérieure qui se réunissent latéralement pour former en partie haute une ceinture destinée à entourer le bassin de la personne portant la culotte. La culotte comporte en outre une partie formant entre-jambe 4 reliant une partie 5p de l'extrémité basse de la face 3 postérieure et une partie 5a de l'extrémité basse de la face 2 antérieure, de manière à former deux ouvertures destinées à recevoir les jambes de la personne portant la culotte 1.

Une partie 6 de la face 2 antérieure est en tissu chauffant en fibre de carbone ou tout autre matière conductrice de la chaleur. La partie 6 s'étend d'une partie de l'extrémité haute de la face 2 antérieure à la partie 5a de l'extrémité basse de la face 2 antérieure sur une longueur de 13 cm et s'étend sur une longueur de 15 cm dans la direction orthogonale. Le reste de la face 2 antérieure est en tissu en coton biologique. Les dimensions de la partie chauffante sont adaptées aux tailles des culottes pour respecter la proportion tissu chauffant/tissu non chauffant. Cette partie 6 en tissu chauffant est destinée à être en face du pelvis de la personne portant la culotte.

Une partie 7 de la face 3 postérieure est en tissu chauffant. La partie 7 s'étend de toute l'extrémité haute de la face 3 postérieure vers la partie 5p de l'extrémité basse de la face 3 postérieure sur une longueur de 13 cm. Le reste de la face 3 postérieure est en tissu en coton biologique. Les dimensions de la partie chauffante sont adaptées aux tailles des culottes pour respecter la proportion tissu chauffant/tissu non chauffant Cette partie 7 est destinée à être en face de la zone lombaire basse de de la personne portant la culotte.

En particulier, on pourrait réaliser la partie formant ceinture sensiblement en totalité en tissu chauffant.

La culotte 1 comporte une batterie 8 pour alimenter en électricité le tissu chauffant. La face 2 antérieure comporte dans sa partie latérale en tissu en coton biologique une poche pour recevoir la batterie 8. Des câbles relient de manière amovible le tissu chauffant à la batterie 8.

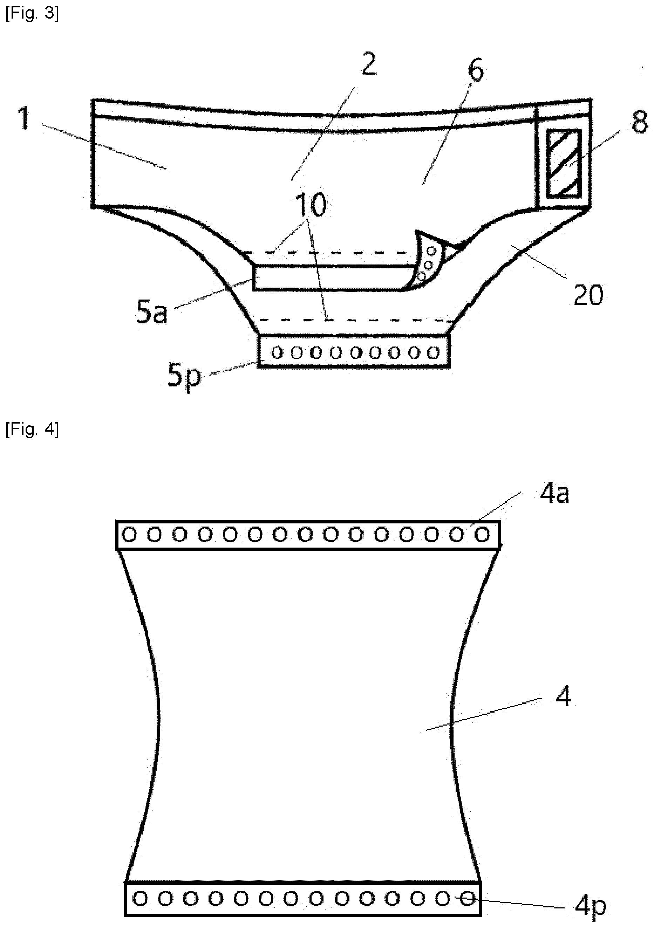

Suivant un mode de réalisation, l'entre-jambe 4 comporte une partie d'extrémité 4a antérieure fixée de manière amovible à la partie 5a de l'extrémité basse de la face 2 antérieure par des boutons poussoirs dont les parties mâles, respectivement femelles, se trouvent sur la partie d'extrémité 4a antérieure tandis que les parties femelles, respectivement mâles, se trouvent sur la face tournée vers l'intérieur (c'est-à-dire vers le porteur à l'état porté de la culotte) de la partie 5a d'extrémité basse , et une partie 4p d'extrémité postérieure fixée de manière amovible à la partie 5p de l'extrémité basse de la face 3 postérieure par des boutons poussoirs dont les parties mâles, respectivement femelles, se trouvent sur la partie d'extrémité 4p antérieure, du même côté de l'entrejambe 4 que les boutons de la partie d'extrémité 4a antérieure, tandis que les parties femelles, respectivement mâles, se trouvent sur la face tournée vers l'intérieur de la partie 5p d'extrémité basse. Deux bandeaux 11 de protection (non représentés à la figure 3) peuvent recouvrir les parties femelles, respectivement mâles, des parties 5a et 5p du côté intérieur de la culotte 1. Ces bandeaux 11 de protection sont cousus le long de lignes 10 de couture pour former un rabat que l'on peut rabattre sur les parties femelles, respectivement mâles, une fois celles-ci entrées en coopération avec les parties mâles, respectivement femelles issus de l'entre-jambe 4. À la place des boutons poussoirs, on pourrait prévoir tout autre type de fixation amovible, notamment une fermeture à glissière, des boutons, des auto agrippants à crochets et boucles (communément appelés Velcro, marque déposée) et analogues.

La partie 4 formant entrejambe n'est pas en tissu chauffant, notamment n'est donc pas en le même tissu que la partie formant ceinture réalisée en tissu chauffant. En particulier, la partie 4 entrejambe est en un matériau absorbant, et, de préférence, est lavable et réutilisable.

En particulier, la partie formant ceinture est en un premier matériau, notamment un premier tissu, ayant une première capacité d'absorbance des liquides, notamment de l'eau, et la partie formant entrejambe est en un deuxième matériau, notamment un deuxième tissu, ayant une deuxième capacité d'absorbance des liquides, notamment de l'eau, la deuxième capacité d'absorbance étant supérieure à la première capacité d'absorbance.

Les capacités d'absorbance peuvent notamment être déterminées par le test AATCC79, édition 2018, défini par l'American Association of Textile Chemists and Colorists.

On peut également utiliser tout autre test, notamment tout test consistant à peser l'échantillon à tester, à l'immerger pendant un certain temps, par exemple 1 minute, dans un bac à eau, à une température de 20°C, puis, après égouttement, à le peser de nouveau, la capacité d'absorbance étant alors définie par le rapport des poids après sur poids avant immersion en pourcentage, un matériau peu ou pas absorbant ayant une capacité sensiblement égale à 1.

## Revendications

1. Culotte (1) menstruelle comportant une partie formant ceinture, ayant une face (2) antérieure et une face (3) postérieure, et une partie (4) amovible formant entrejambe, fixée de manière amovible à la partie formant ceinture de manière à former avec celle-ci deux trous pour le passage des jambes, **caractérisée en ce que** au moins une partie (6 ; 7 ; 6, 7) de la partie formant ceinture est en tissu chauffant, tandis que la partie (4) amovible formant entrejambe n'est pas en tissu chauffant.

2. Culotte (1) suivant la revendication 1, **caractérisée en ce qu'**une batterie (8) est fixée à la culotte de manière à alimenter en électricité le tissu chauffant de la partie (6 ; 7 ; 6, 7) en tissu chauffant.

3. Culotte (1) suivant la revendication 2, **caractérisée en ce que** la batterie (8) est fixée à la partie formant ceinture, notamment à la face (2) antérieure.

4. Culotte (1) suivant la revendication 1, 2 ou 3, **caractérisée en ce que** la partie (4) amovible s'étend entre les faces (2, 3) antérieure et postérieure.

5. Culotte (1) suivant la revendication 4, **caractérisée en ce que** la partie (4) formant entrejambe est fixée d'une part à une extrémité basse de la face (2) antérieure et d'autre part à une extrémité basse de la face (3) postérieure.

6. Culotte (1) suivant l'une des revendications 1 à 5, **caractérisé en ce que** la partie (6 ; 6, 7) en tissu chauffant s'étend, à l'état porté de la culotte, en face du pelvis de la personne portant la culotte.

7. Culotte (1) suivant l'une des revendications 1 à 6, **caractérisé en ce que** la partie (7 ; 6, 7) en tissu chauffant s'étend, à l'état porté de la culotte, en face de la zone lombaire basse de la personne portant la culotte.

8. Culotte (1) suivant l'une des revendications 1 à 7, **caractérisé en ce que** la partie (4) formant entrejambe est en un matériau, notamment en tissu, au moins en partie absorbant, notamment est sensiblement en totalité en un matériau absorbant.

9. Culotte (1) suivant la revendication 8, **caractérisée en ce que** la partie formant ceinture est en un matériau, notamment en tissu, moins absorbant que le matériau absorbant de la partie (4), notamment la partie formant ceinture est en un matériau, notamment en tissu, qui n'est pas absorbant.

10. Culotte (1) suivant l'une des revendications précédentes, **caractérisée en ce que** la partie (4) formant entre-jambe est fixée de manière amovible par des moyens de fixation amovible comportant au moins une fermeture à glissière, notamment deux fermetures à glissière, et/ou des boutons poussoirs à éléments mâles et femelles, et/ou une fermeture par auto agrippants à crochets et boucles.

11. Culotte (1) suivant la revendication 10, **caractérisée en ce que** la culotte, notamment la partie formant ceinture, comporte au moins un bandeau de protection de la au moins une fermeture à glissière et/ou des boutons poussoirs et/ou des auto agrippants du côté intérieur de la culotte, pour éviter l'irritation de l'utilisatrice, notamment sous la forme d'un ou de rabat(s) fixés, notamment cousu(s) ou soudé(s), à au moins l'une des faces antérieure et/ou postérieure.

12. Culotte (1) suivant l'une des revendications 1 à 11, **caractérisé en ce que** la partie (4) formant entre-jambe est un élément jetable.

13. Culotte (1) suivant l'une des revendications 1 à 11, **caractérisé en ce que** la partie (4) formant entre-jambe est un élément lavable et réutilisable.

## Patentansprüche

1. Menstruationsunterhose (1) umfassend ein den Bund bildendes Teil mit einer Vorderseite (2) und einer Rückseite (3), und ein abnehmbares, den Schritt bildendes Teil (4), das abnehmbar an dem den Bund bildenden Teil befestigt ist, sodass es mit diesem zwei Öffnungen für den Durchtritt der Beine bildet, **dadurch gekennzeichnet, dass** mindestens ein Teil (6; 7; 6, 7) des den Bund bildenden Teils aus beheizbarem Gewebe besteht, während das abnehmbare, den Schritt bildende Teil (4) nicht aus beheizbarem Gewebe besteht.

2. Unterhose (1) nach Anspruch 1, **dadurch gekennzeichnet, dass** ein Akku (8) an der Unterhose befestigt ist, um das beheizbare Gewebe des Teils (6; 7; 6, 7) aus beheizbarem Gewebe mit Strom zu versorgen.

3. Unterhose (1) nach Anspruch 2, **dadurch gekennzeichnet, dass** das Akku (8) an dem den Bund bildenden Teil, insbesondere an der Vorderseite (2), befestigt ist.

4. Unterhose (1) nach Anspruch 1, 2 oder 3, **dadurch gekennzeichnet, dass** sich das abnehmbare Teil (4) zwischen der Vorder- und der Rückseite (2, 3) erstreckt.

5. Unterhose (1) nach Anspruch 4, **dadurch gekennzeichnet, dass** das den Schritt bildende Teil (4) einerseits an einem unteren Ende der Vorderseite (2) und andererseits an einem unteren Ende der Rückseite (3) befestigt ist.

6. Unterhose (1) nach einem der Ansprüche 1 bis 5, **dadurch gekennzeichnet, dass** sich das Teil (6; 6, 7) aus beheizbarem Gewebe beim Tragen der Unterhose vor dem Becken der Person, die die Unterhose trägt, erstreckt.

7. Unterhose (1) nach einem der Ansprüche 1 bis 6, **dadurch gekennzeichnet, dass** sich das Teil (7; 6, 7) aus beheizbarem Gewebe beim Tragen der Unterhose vor dem unteren Lendenbereich der Person, die die Unterhose trägt, erstreckt.

8. Unterhose (1) nach einem der Ansprüche 1 bis 7, **dadurch gekennzeichnet, dass** das den Schritt bildende Teil (4) aus einem Material, insbesondere einem Gewebe, besteht, das mindestens teilweise saugfähig ist, insbesondere im Wesentlichen vollständig aus saugfähigem Material besteht.

9. Unterhose (1) nach Anspruch 8, **dadurch gekennzeichnet, dass** das den Bund bildende Teil aus einem Material, insbesondere einem Gewebe, besteht, das weniger saugfähig als das saugfähige Material des Teils (4) ist, insbesondere besteht das den Bund bildende Teil aus einem Material, insbesondere einem Gewebe, das nicht saugfähig ist.

10. Unterhose (1) nach einem der vorstehenden Ansprüche, **dadurch gekennzeichnet, dass** das den Schritt bildende Teil (4) durch Mittel zur abnehmbaren Befestigung, die mindestens einen Reissverschluss, insbesondere zwei Reissverschlüsse, und/oder Druckknöpfe mit männlichen und weiblichen Elementen und/oder einen Klettverschluss mit Haken und Schlaufen umfassen, abnehmbar befestigt ist.

11. Unterhose (1) nach Anspruch 10, **dadurch gekennzeichnet, dass** die Unterhose, insbesondere das den Bund bildende Teil, mindestens einen Abdeckstreifen des mindestens einen Reissverschlusses und/oder der Druckknöpfe und/oder der Klettverschlüsse auf der Innenseite der Unterhose umfassen, um die Reizung der Trägerin zu vermeiden, insbesondere in Form von einer oder von Lasche(n), die an mindestens einer der Vorder- und/oder Rückseite befestigt, insbesondere genäht oder geschweisst ist (sind).

12. Unterhose (1) nach einem der vorstehenden Ansprüche 1 bis 11, **dadurch gekennzeichnet, dass** das den Schritt bildende Teil (4) ein wegwerfbares Element ist.

13. Unterhose (1) nach einem der Ansprüche 1 bis 11, **dadurch gekennzeichnet, dass** das den Schritt bildende Teil (4) ein waschbares und wiederverwendbares Element ist.

## Claims

1. Menstrual pant (1) comprising a part forming a belt, having an anterior face (2) and a posterior face (3), and a movable part (4) forming a crotch, attached in a movable way to the part forming a belt so as to form with said belt two holes through which the legs can pass, **characterised in that** at least one part (6; 7; 6, 7) of the part forming a belt is made of heating fabric, whereas the movable part (4) forming a crotch is not made of heating fabric.

2. Pant (1) according to claim 1, **characterised in that** a battery (8) is attached to the pant so as to supply the heating fabric of the part (6; 7; 6, 7) made of heating fabric with electricity.

3. Pant (1) according to claim 2, **characterised in that** the battery (8) is attached to the part forming a belt, in particular to the anterior face (2).

4. Pant (1) according to claim 1, 2 or 3, **characterised in that** the movable part (4) extends between the anterior and posterior faces (2, 3).

5. Pant (1) according to claim 4, **characterised in that** the part (4) forming a crotch is attached on the one hand to a bottom end of the anterior face (2) and on the other hand to a bottom end of the posterior face (3).

6. Pant (1) according to any of claims 1 to 5, **characterised in that** the part (6; 6, 7) made of heating fabric extends, when the pant is in the worn state, in front of the pelvis of the person wearing the pant.

7. Pant (1) according to any of claims 1 to 6, **characterised in that** the part (7; 6, 7) made of heating fabric extends, when the pant is in the worn state, in front of the lower lumbar area of the person wearing the pant.

8. Pant (1) according to one of claims 1 to 7, **characterised in that** the part (4) forming a crotch is made of a material, in particular fabric, that is at least partially absorbent, in particular substantially entirely made of an absorbent material.

9. Pant (1) according to claim 8, **characterised in that** the part forming a belt is made of a material , in particular fabric, that is less absorbent than the absorbent material of the part (4), in particular the part forming a belt is made of a material, in particular fabric, that is not absorbent.

10. Pant (1) according to any of the preceding claims, **characterised in that** the part (4) forming a crotch is attached in a movable manner by movable attachment means comprising at least one zip fastener, in particular two zip fasteners, and/or press studs with male and female elements, and/or a self-gripping fastener with hooks and loops.

11. Pant (1) according to claim 10, **characterised in that** the pant, in particular the part forming a belt, comprises at least one protective strip for the at least one zip fastener and/or press studs and/or self-gripping fasteners on the inside of the pant, to avoid irritating the user, particularly in the form of one or more flap(s) attached, in particular sewn or bonded, to at least one of the anterior and/or posterior faces.

12. Pant (1) according to one of claims 1 to 11, **characterised in that** the part (4) forming a crotch is a disposable element.

13. Pant (1) according to one of claims 1 to 11, **characterised in that** the part (4) forming a crotch is a washable and reusable element.
